# EUROPÄISCHE PATENTSCHRIFT

(11) **EP 2 699 235 B1**
(45) Veröffentlichungstag und Bekanntmachung des Hinweises auf die Patenterteilung: **11.01.2017**
(21) Anmeldenummer: 12716255.0
(22) Anmeldetag: 19.04.2012
(51) Int. Cl.: A61K 9/16, B29B 9/06, A61K 9/14

(54) **VERFAHREN ZUR HERSTELLUNG VON PHARMAZEUTISCHEN ERZEUGNISSEN AUS EINEM SCHMELZEMATERIAL**
METHOD FOR PRODUCING PHARMACEUTICAL PRODUCTS FROM A MELT MATERIAL
PROCÉDÉ DE FABRICATION DE PRODUITS PHARMACEUTIQUES À PARTIR D'UNE MATIÈRE FONDUE

(30) Priorität: 21.04.2011 DE 102011018403
(43) Veröffentlichungstag der Anmeldung: 26.02.2014
(73) Patentinhaber: Maag Automatik GmbH, 63762 Großostheim (DE)
(72) Erfinder: MÜRB, Reinhardt-Karsten, 63743 Aschaffenburg (DE)
(74) Vertreter: Spranger, Stephan
(86) Internationale Anmeldenummer: PCT/EP2012/001703
(87) Internationale Veröffentlichungsnummer: WO 2012/143133

(56) Entgegenhaltungen:
- EP-A1- 0 544 144
- WO-A1-2011/131344

## Beschreibung

Die Erfindung betrifft ein Verfahren zur Herstellung von pharmazeutischen Erzeugnissen aus einem Schmelzematerial gemäß dem Oberbegriff des Anspruchs 1.

Schmelzematerial allgemein wird heute beispielsweise durch Granulierung be- und verarbeitet. Bei der Granulierung von Schmelzematerial, bisher insbesondere z.B. von Kunststoffen, kommen generell häufig Extruder oder Schmelzepumpen zum Einsatz. Diese Extruder oder Schmelzepumpen drücken geschmolzenes Kunststoffausgangsmaterial durch Düsen einer Lochplatte in ein Kühlmedium, z.B. Wasser. Dabei wird das aus den Öffnungen der Düsen austretende Material von einer Messeranordnung mit zumindest einem umlaufenden Messer dort abgetrennt, so dass Granulatkörner entstehen. Entsprechende Vorrichtungen, welche beispielsweise Verfahren zur Unterwassergranulierung ausführen, sind als Unterwassergranulierungsanlagen beispielsweise unter der Produktbezeichnung SPHERO® des Unternehmens Automatik Plastics Machinery GmbH bekannt.

Die deutsche Offenlegungsschrift DE 10 2009 006 123 A1 der gleichen Anmelderin beschreibt ein Verfahren und eine Vorrichtung zum Granulieren von thermoplastischem Kunststoffmaterial, wobei eine strömungsoptimierte radiale Zuströmung eines Kühlfluids vorgesehen ist, um so den energetischen Aufwand für den Messerantrieb im Kühlfluid zu verringern. Spezielle Lösungen der Problematiken der Herstellung pharmazeutischer Erzeugnisse unter Einbeziehung einer entsprechenden Gestaltung sind dort nicht thematisiert.

So kommt es bei der Herstellung von pharmazeutischen Erzeugnissen aus einem Schmelzematerial wesentlich auf die gleichmäßige Größe und somit Gewicht sowie auf die gleichmäßig erzielbare Formgebung der Erzeugnisse an. Auch sind große Stückzahlen gewünscht, was es erforderlich macht, dass ein entsprechendes Produktionsverfahren zuverlässig bei einer sehr großen Anzahl an Granulatkörnern (z.B. bis zu 50 Millionen Stück pro Stunde) abläuft.

Die deutsche Offenlegungsschrift DE 41 38 513 A1 beschreibt eine feste pharmazeutische Retardform, bei welcher die Formgebung nach der Extrusion einer entsprechenden Schmelzezusammensetzung aus einem Extruder und eine Düsenplatte durch sogenannten Heißabschlag erfolgt, wobei beispielsweise kugelförmige Teilchen erhalten werden sollen. Über die Umsetzbarkeit der dort nur beispielhaft beschriebenen Herstellung bei großen Stückzahlen von herzustellenden Granulatkörnern unter realen Produktionsbedingungen schweigt dieses Dokument allerdings.

Anlagen zur Durchführung des Heißabschlags in Luft als Kühlmedium gibt es schon sehr lange im Markt, da sie verhältnismäßig einfach zu konstruierende Maschinen zum Granulieren von strangextrudierten Thermoplasten darstellen. Dabei werden aus der Lochplatte austretende Schmelzestränge durch dichtest möglich an der Oberfläche rotierende Messer und durch die dem Strangmaterial innewohnende Trägheit in kleine Stücke zu Granulatkörnern zerhackt. Durch die Messerrotation wird aus der Umgebung bzw. dem Inneren des Gehäuses Luft angesaugt, welche die Granulatkörner mehr oder weniger frei und zentrifugal vom Schnittort wegleitet. Die dabei auftretenden Probleme liegen in der schlechten Kühlung der Messer, die mit der Zeit überhitzen und verkleben können, sowie der Neigung zum allgemeinen Verkleben und Verstopfen solcher Anlagen insbesondere bei höheren Durchsätzen mit großen Stückzahlen von herzustellenden Granulatkörnern unter realen Produktionsbedingungen. Weiter neigen so hergestellte Granulatkörner, zumal wenn die Viskosität des Schmelzematerials relativ hoch ist, zu zylindrischen und irregulären Formen, wobei gerade bei pharmazeutischen Materialien in den Folgeanwendungen eher sehr viele kugelförmige Granulatkörner einheitlicher Größe benötigt werden.

Die europäische Patentanmeldung EP 0 544 144 A1 beschreibt eine pharmazeutische Retardform, welche unter anderem allgemein durch Heißabschlag herstellbar sein kann.

Die Schrift WO 2011/131344 A1 beschreibt allgemein eine Vorrichtung und ein Verfahren zur Herstellung von Granulatkörnern.

Es ist die Aufgabe der vorliegenden Erfindung, ein Verfahren zur Herstellung von pharmazeutischen Erzeugnissen aus einem Schmelzematerial vorzusehen, welches die Nachteile des Standes der Technik überwindet und insbesondere auf relativ einfache und kostengünstige Weise eine effektive Granulierung von Granulatkörnern pharmazeutischer Produkte mit gleichmäßiger Korngröße und gleichmäßiger sowie gleichbleibender Formgebung auch bei großen Stückzahlen von herzustellenden Granulatkörnern unter realen Produktionsbedingungen ermöglicht.

Diese Aufgabe wird erfindungsgemäß gelöst mit einem Verfahren mit den Merkmalen gemäß Anspruch 1. Bevorzugte Ausführungsformen der Erfindung sind in den abhängigen Ansprüchen definiert.

Bei dem erfindungsgemäßen Verfahren zur Herstellung von pharmazeutischen Erzeugnissen aus einem Schmelzematerial, tritt das Schmelzematerial aus Düsen in einer Lochplatte aus und wird dann granuliert, wobei eine Messeranordnung mit zumindest einem Messer, angetrieben von einem Motor, der Lochplatte gegenüberliegend angeordnet wird, so dass das zumindest eine Messer die Düsen in der Lochplatte überstreicht und dabei Granulatkörner des austretenden Schmelzematerials abtrennt, wobei ein Gehäuse vorgesehen wird, welches an die Lochplatte anschließt, zumindest das zumindest eine Messer der Messeranordnung umgibt und von einem Kühlmedium durchströmt wird, so dass dabei die Granulatkörner aus dem Schmelzematerial in dem Kühlmedium verfestigt werden, welches aus einer Zuströmeinrichtung, gebildet von einer separaten Zuströmkammer, welche das Gehäuse im Rotationsbereich des zumindest einen Messers umfänglich umgibt, und einer umfänglich verlaufend angeordneten Zuströmdüsenanordnung zwischen der Zuströmkammer und dem Gehäuse umfänglich von allen Seiten radial von außen nach innen, d.h. zentripetal, oder im Wesentlichen radial von außen nach innen in das Gehäuse eingeleitet wird, wobei zumindest im Rotationsbereich eine zentripetale oder zumindest im Wesentlichen zentripetale Strömung des Kühlmediums ausgebildet wird und im Weiteren das Kühlmedium und die darin befindlichen Granulatkörner einem Auslass im Gehäuse zugeführt werden, wobei das Kühlmedium ein gasförmiges Kühlmedium ist. Bei dem erfindungsgemäßen Verfahren wird so durch die entsprechend gestaltete Zuströmkammer und durch die Zuströmdüsenanordnung und/oder mittels einer oder mehrerer Leiteinrichtung(en) eine umfänglich gleichmäßige, d.h. über den Umfang gleich bleibend große oder zumindest im Wesentlichen gleich bleibend große, Durchsatzmenge an gasförmigem Kühlmedium, z.B. Luft oder ein Inertgas wie etwa Stickstoff oder ein Reaktionsgas, welches so ausgewählt ist, dass es mit dem zu granulierenden pharmazeutischen Schmelzematerial eine gewünschte chemische Reaktion eingehen kann, vorgesehen, welche entsprechend in dem Rotationsbereich in das Gehäuse von allen Seiten radial von außen nach innen strömend eingeleitet wird. Gemäß dem erfindungsgemäßen Verfahren beträgt das Verhältnis des Massestroms des gasförmigem Kühlmediums zum Massestrom der sich darin befindenden Granulatkörner im Gehäuse ein Beladungsverhältnis, definiert als Masse der Granulatkörner pro Stunde zu Masse des gasförmigen Kühlmediums pro Stunde, im Bereich von 0,3 bis 0,7. Das Verkleben von Granulatkörnern kann somit auch bei hohen Durchsatzmengen besonders zuverlässig vermieden werden, da ausreichend Kühlmedium vorhanden ist, um die Granulatkörner ohne Verklumpen einzeln zu umgeben und so zu kühlen und zu transportieren. Erfindungsgemäß wird somit das zur Kühlung und zum Abtransport der frisch abgetrennten Granulatkörner benötigte, speziell im Hinblick auf die üblicher Weise vorhandene Feuchtigkeitsempfindlichkeit von pharmazeutischen Materialien gasförmige Kühlmedium bzw. Kühlfluid so dem Gehäuse der entsprechenden Granuliervorrichtung zugeführt, dass es dem zumindest einen Messer der Messeranordnung möglichst wenig Widerstand entgegensetzt und gleichzeitig die Granulatkörner aus dem pharmazeutischen Schmelzematerial schnellstmöglich aus dem Rotationsbereich und somit dem Abtrennbereich entfernt werden. Somit ist ein hoher spezifischer Durchsatz an Material (großen Stückzahlen relativ kleiner Granulatkörnern) möglich, wobei gleichzeitig ein Verklumpen der Granulatkörner durch die erfindungsgemäß gute Kühlung und das erfindungsgemäß erreichbare gleichmäßige Strömungsverhalten des gasförmigen Kühlmediums mit den darin sich befindenden Granulatkörnern aus dem pharmazeutischen Schmelzematerial vermeidbar ist.

Das gasförmige Kühlmedium wird über die umfänglich verlaufend angeordnete Zuströmdüsenanordnung von außen nach innen, d.h. zentripetal, oder im Wesentlichen von außen nach innen dem Gehäuse im Rotationsbereich, also im Bereich der Schnittebene, erfindungsgemäß zugeführt. Diese Zuströmdüsenanordnung wird über die um das Gehäuse herumlaufend angeordnete separate Zuströmkammer gespeist. Durch die entsprechend vorgesehene Gestaltung der Zuströmeinrichtung und/oder Festlegung der Abmessungen der Zuströmdüsenanordnung und/oder mittels der einen oder mehreren Leiteinrichtung(en) kann dem gasförmigen Kühlmedium bei Eintritt in das Gehäuse bzw. bei Eintritt in die Schneidkammer auch eine (zusätzliche) Umlaufgeschwindigkeit verliehen werden, die in etwa der Rotationsgeschwindigkeit des zumindest einen Messers der Messeranordnung entspricht. Die dabei erfolgende Beschleunigung des gasförmigen Kühlmediums auf die gewünschte Geschwindigkeit, d.h. die zur Erreichung des entsprechenden Drehimpulses benötigte Energie, kann dem Druck des gasförmigen Kühlmediums entnommen werden. Die zusätzliche Umlaufgeschwindigkeit des gasförmigen Kühlmediums, welche oben zusätzlich vorgesehen sein kann, kann entweder mechanisch über die Gestaltung der Zuströmdüsenanordnung und/oder über die Steuerung der Durchsatzmenge des gasförmigen Kühlmediums eingestellt und an unterschiedliche sonstige Verfahrensparameter (Materialdurchsatz, zu granulierendes Schmelzematerial, Größe der Granulatkörner u.Ä.) angepasst werden. Auch die Anzahl und Drehzahl des/der Messer kann entsprechend angepasst werden.

Da erfindungsgemäß das gasförmige Kühlmedium in etwa mit derselben Geschwindigkeit wie die Rotationsgeschwindigkeit des zumindest einen Messers im Rotationsbereich zuströmen kann, wird es das zumindest eine Messer bzw. gegebenenfalls ein Zwischenraum zwischen mehreren Messern der Messeranordnung durchfluten und die frisch abgetrennten Granulatkörner aus dem Rotationsbereich mit sich abführen, was ein Verkleben der Granulatkörner auch bei höheren Durchsatzmengen zuverlässig verhindern kann. Bei der sich ergebenden Strömung wird sich bei Annäherung an die Achse der Rotation des zumindest einen Messers der Messeranordnung die entsprechende Umlaufgeschwindigkeit des gasförmigen Kühlmediums erhöhen und somit sich die entsprechende Zentrifugalkraft erhöhen, so dass die Strömungsbewegung von außen nach innen hin zunehmend erschwert wird und letztlich verhindert wird. Somit wird das gasförmige Kühlmedium in den Raum hinter dem zumindest einen Messer der Messeranordnung strömen und dabei in einer wendelförmigen Strömung von dem Bereich der Lochplatte und dem Rotationsbereich in dem Gehäuse wegströmen.

Bei dem erfindungsgemäßen Verfahren kann also dem Kühlmedium, welches dem Gehäuse zuströmt, mittels der Formgebung der Zuströmkammer und der Zuströmdüsenanordnung und/oder mittels einer oder mehrerer Leiteinrichtung(en) im Bereich der Zuströmdüsenanordnung im Rotationsbereich die zentripetale oder zumindest im Wesentlichen zentripetale Strömung des Kühlmediums aufgeprägt werden und bevorzugt auch ein zusätzlicher Drehimpuls aufgeprägt werden, welcher entsprechend der Richtung der Rotation des zumindest einen Messers ausgerichtet ist.

Bevorzugt kann die Größe des zusätzlichen Drehimpulses dabei so groß sein, dass die entsprechende Geschwindigkeit des gasförmigen Kühlmediums in Richtung der Rotation der Messeranordnung so groß ist wie die Rotationsgeschwindigkeit der Messeranordnung. Somit kann eine weiter optimierte Strömungsführung des Kühlmediums, wie oben erläutert, bei dieser Ausführungsform des erfindungsgemäßen Verfahrens ermöglicht werden. Die Strömung des gasförmigen Kühlmediums verläuft dabei bevorzugt so, dass sie sich senkrecht zur Lochplatte aufrichtet und wegströmt. Dort entstehende Granulatkörner werden daher senkrecht bis wendelförmig von der Lochplatte weggeblasen. Der Volumenstrom des erfindungsgemäß strömenden gasförmigen Kühl- und Transportmediums wird dabei zweckmäßig so gewählt, dass die Granulatkörner sofort nach dem Schnitt vereinzelt werden, also im großen Überschuss.

Beispielsweise treten je Stunde 4 kg Polymer/pharmazeutisches Schmelzematerial von 1 200 kg/m³ Dichte aus einer Lochplatte mit 24 Löchern und einem Teilkreisdurchmesser d_{Lp} von rund 60 mm aus und werden durch 9 Messer mit n = 3 900 1/min in 13 900 Granulatkörner pro Sekunde mit 0,5 mm Durchmesser zerteilt. Die Granulatkörner sollen in jeder Richtung einen Abstand a von etwa 1 cm zueinander haben. Der Massenstrom des gasförmigen Kühl- und Fördermediums beträgt dabei etwa 8 kg/h und trägt dabei 4 kg/h Fördergut, was einem Verhältnis Fördergut zu Fördermedium ("Beladung") von 0,5 entspricht. Dies ist weit weniger als in der pneumatischen Förderung üblich, wo selbst bei Flugförderung ein Beladungsverhältnis von 10 bis 20, bei der Dickstromförderung von 60 und darüber üblich ist. Demgegenüber wird also die Kühl- und Transportluft im großen Überschuss zugeführt.

Betrachtet man die auftretenden Wärmeströme, so ist feststellbar, dass je nach Polymer/pharmazeutischem Schmelzematerial bei Zufuhr von z.B. 20°C warmer Luft eine Endtemperatur von Luft und darin befindlichen Granulatkörnern von etwa 55 °C erreicht wird. Für intensivere oder noch schnellere Kühlung müsste also die Luftmenge erhöht oder die Zufuhrtemperatur weiter gesenkt werden.

Auch kann bei dem erfindungsgemäßen Verfahren eine Durchsatzmenge und/oder ein Druck und/oder eine Richtung des über die Zuströmeinrichtung zugeführten gasförmigen Kühlmediums mittels einer Steuereinrichtung so gesteuert werden, dass dadurch eine Richtung der Strömung des Kühlmediums in das Gehäuse eingestellt wird. Zum Beispiel kann die Steuereinrichtung die eine oder mehreren Leiteinrichtung(en) aufweisen bzw. steuern.

Bevorzugt kann gemäß dem erfindungsgemäßen Verfahren also das Verhältnis des Massestroms des gasförmigem Kühlmediums zum Massestrom der sich darin befindenden Granulatkörner im Gehäuse ein Beladungsverhältnis, definiert als Masse der Granulatkörner pro Stunde zu Masse des gasförmigen Kühlmediums pro Stunde, von 0,5 betragen.

Erfindungsgemäß bevorzugt können die Granulatkörner, welche sich in dem gasförmigen Kühlmedium befinden, nach dem Rotationsbereich weiter dem Bereich des Auslasses des Gehäuses zuströmen, in welchem sie in einem Winkel von weniger als 10° gegen eine dortige Wandung des Gehäuses geführt werden, so dass den sich in dem gasförmigen Kühlmedium befindlichen Granulatkörnern dort eine rollende Bewegung aufgeprägt wird. Somit kann erfindungsgemäß bevorzugt die gleichmäßige Formgebung der Granulatkörner besonders zuverlässig erreichbar sein.

Die Verfestigung der Granulatkörner kann dabei zusätzlich unterstützt werden, indem die Wandung des Gehäuses gekühlt wird, zum Beispiel in doppelwandiger Ausführung von Kühlfluid durchströmt wird.

Zur weiteren Strömungsoptimierung auch im Bereich des Auslasses kann der Auslass in dem der Zuströmeinrichtung abgewandten Bereich des Gehäuses der erfindungsgemäßen Vorrichtung angeordnet sein. Somit kann ein gleichmäßiges Abströmen des gasförmigen Kühlmediums mit den sich darin befindenden Granulatkörnern aus dem pharmazeutischen Schmelzematerial erreicht werden, wodurch auch ein eventuelles Verklumpen im Gehäuse und insbesondere im Bereich des Auslasses zusätzlich besonders zuverlässig vermieden werden kann. Dabei können die Granulatkörner beispielsweise in einer Auslaufspirale gesammelt und tangential vom Gehäuse weggeführt werden.

Die Erfindung wird im Folgenden beispielhaft anhand der beigefügten Figur sowie anhand der angegebenen Beispiele näher erläutert werden. Es zeigt:

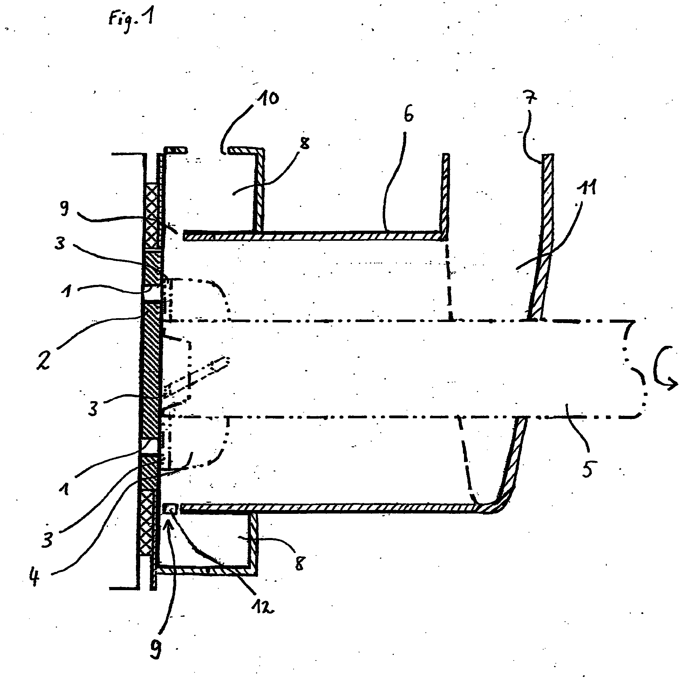

- Fig. 1: in einer schematischen Schnittansicht eine Granulierungsvorrichtung zur Durchführung des erfindungsgemäßen Verfahrens.

Die Figur 1 zeigt schematisch in einer Schnittansicht eine Vorrichtung zum Granulieren von aus Düsen 1 in einer Lochplatte 2 austretendem pharmazeutischem Schmelzematerial.

Die in Fig. 1 schematisch gezeigte Granulierungsvorrichtung weist eine Lochplatte 2 mit darin vorgesehenen Düsen 1 auf, wobei die Anordnung der Düsen 1 im Wesentlichen rotationssymmetrisch ist und auch die sonstige Gestaltung der Vorrichtung rotationssymmetrisch bzw. im Wesentlichen rotationssymmetrisch ist. Gemäß der Darstellung der Fig. 1 ist der Lochplatte 2 eine Messeranordnung zugeordnet mit zumindest einem Messer 3, welches von einem Messerträger 4, angeordnet auf einer Messerwelle 5, gebildet ist. Die Messeranordnung wird von einem Motor (in Fig. 1 nicht gezeigt) angetrieben, so dass das zumindest eine Messer 3 die Düsen 1 in der Lochplatte 2 überstreicht und dabei Granulatkörner des aus den Düsen 2 austretenden pharmazeutischen Schmelzematerials abtrennt. Das pharmazeutische Schmelzematerial kann auf herkömmliche Art und Weise geschmolzen werden und zum Beispiel über einen Extruder oder eine Schmelzepumpe (in Fig. 1 nicht gezeigt) zu dem Bereich der Lochplatte 2 transportiert werden und aus den Düsen 1 dort gedrückt werden. Die Vorrichtung weist ein Gehäuse 6 auf, welches an die Lochplatte 2 anschließt und so eine Schneidkammer definiert, welche im Betrieb erfindungsgemäß mit einem gasförmigen Kühlmedium, üblicherweise z.B. Luft, gefüllt und durchströmt ist, wobei das Gehäuse 6 zumindest das eine Messer 3 und den Messerträger 4 sowie zumindest einen Teil der Messerwelle 5 umgibt. Die Messerwelle 5 ist in dem Gehäuseteil, welches der Lochplatte 2 abgewandt ist, fluiddicht aus dem Gehäuse herausgeführt, und der Motor (in Fig. 1 nicht gezeigt) ist vorgesehen, welcher über die Messerwelle 5 das zumindest eine Messer 3 zu einer Rotationsbewegung antreibt. Es ist eine Zuströmeinrichtung vorgesehen mit einer separaten Zuströmkammer 8, welche das Gehäuse 6 im Bereich der Rotation des zumindest einen Messers 3 umfänglich umgibt, und mit einer umfänglich verlaufend angeordneten Zuströmdüsenanordnung 9 zwischen der Zuströmkammer 8 und dem Gehäuse 6, wobei die Zuströmdüsenanordnung 9 im in der Fig. 1 gezeigten Fall eine umfänglich umlaufende Ringspaltdüse mit einer über den Umfang gleichbleibenden Düsenbreite von z.B. 3 mm ist. Die Zuströmkammer 8 weist erfindungsgemäß über deren Umfang, d.h. umfänglich, von einer Einlassöffnung 10 für das Kühlmedium in der Zuströmkammer 8 beginnend in Rotationsrichtung des zumindest einen Messers 3 einen abnehmenden Querschnitt auf.

Gemäß der in Fig. 1 gezeigten Gestaltung sind mehrere Leitvorrichtungen 12 vorgesehen, so dass durch die Zuströmdüsenanordnung 9 eine umfänglich gleichmäßige Durchsatzmenge an gasförmigem Kühlmedium strömt. Somit wird erfindungsgemäß durch die Zuströmdüsenanordnung 9 zwischen der Zuströmkammer 8 und dem Gehäuse 6 das gasförmige Kühlmedium umfänglich von allen Seiten radial von außen nach innen oder im Wesentlichen radial von außen nach innen in das Gehäuse 6 eingeleitet. Dabei ergibt sich zumindest im Rotationsbereich des zumindest einen Messers 3 eine zentripetale oder zumindest im Wesentlichen zentripetale Strömung des gasförmigen Kühlmediums. Die Leiteinrichtungen 12 sind so angeordnet, dass in Umfangsrichtung immer noch eine Möglichkeit gegeben ist, dass das gasförmige Kühlmedium in alle Bereiche der Zuströmkammer 8 strömen kann. Die Leiteinrichtungen 12 dienen dabei der Strömungsführung des gasförmigen Kühlmediums und nicht dazu, einzelne Bereiche über den Umfang der separaten Zuströmkammer 8 abzuteilen. Die Anordnung der einzelnen Leiteinrichtungen 12 kann beispielsweise gleichmäßig über den Umfang der Zuströmkammer 8 bzw. der Zuströmdüsenanordnung 9 verteilt sein. Die Befestigung der einzelnen Leiteinrichtungen 12 kann ortsfest z.B. durch Verschweißen entsprechender Leitflügel an die Wandungen erfolgen. Die Leiteinrichtung(en) 8 können auch einzeln oder bevorzugt gemeinsam z.B. durch eine Steuereinrichtung verstellbar ausgeführt sein, wobei z.B. der Anstellwinkel entsprechend verstellbar sein kann.

Gemäß der Darstellung der Fig. 1 ist ein Auslass 7 in dem der Zuströmeinrichtung abgewandten Bereich des Gehäuses 6 angeordnet. Nach dem Rotationsbereich strömt das gasförmige Kühlmedium mit den darin sich befindenden Granulatkörnern weiter dem Bereich des Auslasses 7 des Gehäuses 6 zu, in welchem sie in einem Winkel von weniger als 10° gegen eine dortige Wandung des Gehäuses 6 geführt werden, so dass den sich in dem gasförmigen Kühlmedium befindlichen Granulatkörnern aus dem pharmazeutischen Schmelzematerial dort eine rollende Bewegung aufgeprägt wird. Dabei ist gemäß der Darstellung der Fig. 1 zum Auslass 7 hin ein wendelförmiger Auslaufabschnitt 11 vorgesehen, welcher die Strömung des durch den Auslass 7 ausströmenden gasförmigen Kühlmediums mit den darin befindlichen Granulatkörnern entsprechend führt und somit auch einen Druckaufbau in diesem Bereich des Gehäuses 6 und/oder im Auslass 7 ermöglicht, nämlich aufgrund des durch den spiralförmigen Auslaufabschnitt 1 sich ergebenden Staudrucks. Auch ein entsprechender spiralförmiger Auslaufabschnitt ist konstruktiv möglich.

Die in Figur 1 gezeigte Vorrichtung dient der Durchführung des erfindungsgemäßen Verfahrens in der Anwendung für die Herstellung von pharmazeutischen Erzeugnissen bzw. Granulatkörnern aus einem entsprechenden Schmelzematerial.

So wurden bereits Versuche gemäß dem erfindungsgemäßen Verfahren mit einer entsprechenden bzw. ähnlichen Anlage der Anmelderin bei großen Stückzahlen von herzustellenden Granulatkörnern unter realen Produktionsbedingungen (jedoch noch nicht unter in allen Belangen optimierten Verfahrensparametern) vorgenommen. Die Ergebnisse der Versuche mit unterschiedlichen pharmazeutischen Schmelzematerialien sind in der Tabelle 1 zusammengestellt.

Die angegebenen Temperaturen betreffen dabei die Temperaturen der Anlagenbauteile (Extruderheizzonen, Lochplatte, usw.). Die tatsächlich beim Austritt aus der Lochplatte vorhandene Temperatur der Schmelzestränge dürfte um einige Grad höher liegen. Bei allen granulierten pharmazeutischen Schmelzematerialien kam Luft als das erfindungsgemäß gasförmige Kühlmedium zum Einsatz, wobei die Temperaturen der Luft dabei zwischen 15°C und 60°C lagen.

**Tabelle 1:**

| **Material** | **Temperaturen der Düsenplatte und der umgebenden Anlagenteile** | **Form der erzeugten Granulatkörner** |
|---|---|---|
| Hoechst Wachs PE190 | 100°C ... 130°C | Aufgeschäumte Agglomerate |
| Plasdone K12 | 130°C ... 170°C | Flakes beliebiger Form |
| Eudragit RS PO | 140°C ... 180°C | Flakes d≈1,5mm |
| 5/21 Eudragit RL PO + 15/21 Eudragit RS PO + 1/21 CaSt | 140°C ... 170°C | Flakes d≈2mm |
| 10% Paracetamol + 20% PEG + 70% Basewax | 50°C ... 70°C | Zylinderförmig, d≈1mm |
| 10% Paracetamol + 90% CaSt | 100°C ... 140°C | Kugelförmig, d≈1mm |

## Patentansprüche

1. Verfahren zur Herstellung von pharmazeutischen Erzeugnissen aus einem Schmelzematerial,
wobei das Schmelzematerial aus Düsen in einer Lochplatte austritt und dann granuliert wird, **dadurch gekennzeichnet, dass**
eine Messeranordnung mit zumindest einem Messer, angetrieben von einem Motor, der Lochplatte gegenüberliegend angeordnet wird, so dass das zumindest eine Messer die Düsen in der Lochplatte überstreicht und dabei Granulatkörner des austretenden Schmelzematerials abtrennt, wobei ein Gehäuse vorgesehen wird, welches an die Lochplatte anschließt, zumindest das zumindest eine Messer der Messeranordnung umgibt und von einem Kühlmedium durchströmt wird, so dass dabei die Granulatkörner aus dem Schmelzematerial in dem Kühlmedium verfestigt werden, welches aus einer Zuströmeinrichtung, gebildet von einer separaten Zuströmkammer, welche das Gehäuse im Rotationsbereich des zumindest einen Messers umfänglich umgibt, und einer umfänglich verlaufend angeordneten Zuströmdüsenanordnung zwischen der Zuströmkammer und dem Gehäuse umfänglich von allen Seiten radial von außen nach innen oder im Wesentlichen radial von außen nach innen in das Gehäuse eingeleitet wird, wobei zumindest im Rotationsbereich eine zentripetale oder zumindest im Wesentlichen zentripetale Strömung des Kühlmediums ausgebildet wird und im Weiteren das Kühlmedium und die darin befindlichen Granulatkörner einem Auslass im Gehäuse zugeführt werden, wobei das Kühlmedium ein gasförmiges Kühlmedium ist, wobei das Verhältnis des Massestroms des gasförmigem Kühlmediums zum Massestrom der sich darin befindenden Granulatkörner im Gehäuse ein Beladungsverhältnis, definiert als Masse der Granulatkörner pro Stunde zu Masse des gasförmigen Kühlmediums pro Stunde, im Bereich von 0,3 bis 0,7 beträgt.

2. Verfahren nach Anspruch 1, **dadurch gekennzeichnet, dass** das Beladungsverhältnis 0,5 beträgt.

3. Verfahren nach Anspruch 1 oder 2, **dadurch gekennzeichnet, dass** die Granulatkörner, welche sich in dem gasförmigen Kühlmedium befinden, nach dem Rotationsbereich weiter dem Bereich des Auslasses des Gehäuses zuströmen, in welchem sie in einem Winkel von weniger als 10° gegen eine dortige Wandung des Gehäuses geführt werden, so dass den sich in dem gasförmigen Kühlmedium befindlichen Granulatkömern dort eine rollende Bewegung aufgeprägt wird.

4. Verfahren nach einem der Ansprüche 1 bis 3, **dadurch gekennzeichnet, dass** das gasförmige Kühlmedium Luft oder ein Inertgas oder ein Reaktionsgas ist, welches so ausgewählt ist, dass es mit dem zu granulierenden pharmazeutischen Schmelzematerial eine gewünschte chemische Reaktion eingehen kann.

## Claims

1. Method for producing pharmaceutical products from a melt material, wherein the melt material emerges from nozzles in a perforated plate and is then granulated,
**characterized in that**
a motor-driven cutter arrangement having at least one blade is located opposite the perforated plate so that the at least one blade passes over the nozzles in the perforated plate and in so doing cuts pellets of the emerging melt material, wherein a housing is provided that adjoins the perforated plate and encloses at least the at least one blade of the cutter arrangement and through which housing flows a coolant, so that in the process the pellets of the melt material are solidified in the coolant, which is introduced into the housing from an inlet apparatus comprised of a separate inlet chamber that circumferentially encloses the housing in the area of rotation of the at least one blade and of an inlet nozzle arrangement extending circumferentially between the inlet chamber and the housing, the coolant being introduced circumferentially from all sides radially inward from the outside, or essentially radially inward from the outside, wherein a centripetal or at least substantially centripetal flow of the coolant is produced at least in the area of rotation, and in addition the coolant and the pellets located therein are conveyed to an outlet in the housing, wherein the coolant is a gaseous coolant, wherein the ratio in the housing of the mass flow rate of the gaseous coolant to the mass flow rate of the pellets located therein is a loading ratio, defined as the mass of pellets per hour to the mass of gaseous coolant per hour, in the range from 0.3 to 0.7.

2. Method according to claim 1, **characterized in that** the loading ratio is 0.5.

3. Method according to claim 1 or 2, **characterized in that** after the rotation region, the pellets located in the gaseous coolant can flow onward into the region of the housing outlet, where they are directed against a wall of the housing at an angle of less than 10°, so that a rolling motion is imposed on the pellets located in the gaseous coolant there.

4. Method according to any one of claims 1 through 3, **characterized in that** the gaseous coolant is air or an inert gas or a reaction gas, which is selected such that it can enter into a desired chemical reaction with the pharmaceutical melt material to be granulated.

## Revendications

1. Procédé pour la fabrication de produits pharmaceutiques à partir d'une masse fondue, la masse fondue étant extrudée par des orifices agencés dans une plaque perforée et ensuite granulée,
**caractérisé en ce que**
un agencement de lames comportant au moins une lame, entraîné par un moteur disposé en face de la plaque perforée, de sorte que la au moins une lame effleure les orifices de la plaque perforée et ce faisant sépare les granulés de la masse fondue extrudée, un boîtier étant prévu adjacent à la plaque perforée et qui entoure la au moins une lame de l'agencement des lames et est traversé par un fluide réfrigérant de sorte que les granulés provenant de la masse fondue se solidifient, ledit fluide sortant du dispositif d'alimentation constitué par une chambre d'alimentation entourant circonférentiellement la chambre de coupe dans la zone de rotation de la au moins une lame et comportant un agencement d'orifices d'alimentation agencés circonférentiellement autour de la chambre de coupe entre la chambre d'alimentation et le boîtier de sorte que le fluide réfrigérant peut être dirigé circonférentiellement en provenance de tous les côtés radialement de l'extérieur vers l'intérieur ou sensiblement radialement de l'extérieur vers l'intérieur, un écoulement centripète ou au moins sensiblement centripète étant formé au moins dans la zone de rotation et par la suite le fluide réfrigérant et les granulés qui s'y trouvent étant amenés à une sortie du boîtier, le fluide réfrigérant étant un fluide réfrigérant gazeux, le rapport de masse du flux de fluide réfrigérant gazeux sur la masse des granulés qui s'y trouvent dans le boîtier est un rapport de chargement, défini comme la masse des granulés par heure sur la masse du fluide réfrigérant par heure dans une gamme allant de 0,3 à 0,7.

2. Procédé selon la revendication 1, **caractérisé en ce que** le rapport de chargement est de 0,5.

3. Procédé selon la revendication 1 ou 2, **caractérisé en ce que** les granulés qui se trouvent dans le fluide réfrigérant en aval de la zone de rotation sont dirigés vers la sortie du boîtier, dans lequel ils sont amenés contre une paroi du boîtier avec un angle de moins de 10° de sorte qu'un mouvement de roulement est imprimé aux granulés se trouvant dans le fluide réfrigérant.

4. Procédé selon la revendication 1 à 3, **caractérisé en ce que** le fluide réfrigérant est de l'air ou un gaz inerte ou un gaz réactif qui est choisi de telle façon qu'il peut subir une réaction chimique souhaitée avec la substance pharmaceutique de la masse fondue à granuler.
